# EUROPEAN PATENT APPLICATION

(11) **EP 4 227 957 A1**
(43) Date of publication of application: **16.08.2023**
(21) Application number: 22171977.6
(22) Date of filing: 06.05.2022
(51) Int. Cl.: G16H 50/20, G16H 10/40, G16H 30/40, G06T 7/00, G06N 3/08

(54) **METHOD OF PERFORMING LUNG NODULE ASSESSMENT**

(30) Priority: 15.02.2022 EP 22156808
(71) Applicant: Siemens Healthcare GmbH, 91052 Erlangen (DE)
(72) Inventor: Hölzer, Philipp, 91088 Bubenreuth (DE)

(57) **Abstract**

The invention describes a method of performing lung nodule assessment, which method comprises the steps of obtaining a lung scan (PS) for a patient (P) from an imaging modality (21); obtaining a blood panel (BP) for that patient (P) from a blood analysis modality (22); and processing the lung scan (PS) and the blood panel (BP) in a classifier (1), which classifier (1) is trained to assess a lung nodule (N) on the basis of the lung scan (PS) and the blood panel (BP). The invention further describes a method of training such a classifier (1), and a lung nodule assessment arrangement (2).

## Description

It is important to detect lung cancer at an early stage in order for the patient to be treated successfully. When caught early, the prospects of curing lung cancer are considerably higher than at a later stage. Lung cancer can develop from a lung nodule, which may be visible in a lung scan such as a 2D X-ray image, a 3D computed tomography scan, or any scan obtained using techniques such as magnetic resonance imaging, ultrasound, photoacoustic tomography, diffusion-weighted tomography, positron emission tomography, etc.

However, numerous other maladies may also result in the formation of lung nodules. For example, lung nodules may result from mycobacterial infection, fungal infection, round pneumonia, lung abscess, septic emboli, nocardia infection, hydatid cyst or echinococcosis, Q fever from coxiella burnetii, etc. Lung nodules may also arise from immune system disorders such as rheumatoid arthritis, granulomatosis with polyangiitis, nodular sarcoidosis, organizing pneumonia (cryptogenic or secondary), lymphoid granulomatosis, necrotizing sarcoid granulomatosis. Further causes of lung nodules may be congenital abnormalities such as arteriovenous malformation, bronchogenic cysts, pulmonary sequestration, pulmonary venous varix, bronchial atresia with bronchocele; future causes may be rounded atelectasis, endoparenchymal lymph node, progressive mass fibrosis, inflammatory pseudotumor, amyloidosis, lipoid pneumonia, etc.

A pulmonary nodule or lung nodule can be detected in a lung scan such as an X-ray image or a CT image. However, benign nodules are similar in appearance to malignant nodules. It is known to estimate an image-based malignancy score or "risk score" for a pulmonary nodule under consideration of further information about the patient, for example the patient's age, smoking history, etc. Examples of such approaches are the Mayo/Swensen model, the Brock model and the VA (Veterans Administration) model. However, the overwhelming majority of detected pulmonary nodules (80-95%) is benign, so that the estimated risk score is frequently incorrect. In an effort to improve pulmonary nodule classification, it is known to combine image marker information (e.g. markers or features identified from a CT lung scan) and blood marker information (e.g. markers or features such as genomic, epigenomic, transcriptomic, metabolomic or proteomic markers identified in a blood test or blood panel) to obtain a more accurate risk score. However, the accuracy of the known techniques depends on the relevance of the blood marker data as well as on the clinician's ability to evaluate the additional information in the context of the lung nodule. Even with this combined approach, the rate of misdiagnosis is still unacceptably high, leading to false positive diagnosis, but also false negative diagnosis.

A false-positive misdiagnosis (a high risk score is assigned to a lung nodule) often results in follow-up treatment for the patient with further costs, additional exposure to radiation, and possibly also surgical complications. In addition to the significant costs of unnecessary treatment, the patient may needlessly suffer from psychological anxiety. A false-negative misdiagnosis (a low risk score is assigned to a lung nodule) can mean that early-stage lung cancer remains undetected, and this may result in a significantly worse outlook for the patient, particularly for a patient not participating in a regular screening program.

Artificial intelligence (AI) can be applied to analyse vast quantities of data in order to identify relationships that would otherwise be difficult or impossible to detect using conventional "manual" techniques. For example, an AI classifier can be trained to recognise certain type of pattern in input data. A classifier can be realised in various ways, for example as a decision tree, a support-vector machine, a Bayes classifier, a neural network, etc. It is known to use AI to analyse lung scans in order to identify lung nodules. However, because a "harmless" lung nodule is often visually indistinguishable from a malignant carcinoma, the clinical usefulness of such an approach is limited.

It is therefore an object of the invention to improve the accuracy of pulmonary nodule classification.

This object is achieved by the claimed method of performing lung nodule assessment; the claimed method of training a classifier; and the claimed lung nodule assessment arrangement.

According to the invention, the method of performing lung nodule assessment comprises the steps of obtaining a lung scan of a patient from an imaging modality; obtaining a blood panel for that patient; processing the lung scan and the blood panel in an AI classifier such as a residual neural network (ResNet), a convolutional neural network, a Bayes classifier, etc. The classifier is trained to assess a lung nodule on the basis of the lung scan and the blood panel and to output the results of the classification in a meaningful manner, for example as a cancer risk score, as a probability of malignancy, etc.

A lung scan (or "pulmonary scan") can be a 2D image such as an X-ray of the chest cavity, a 3D image such as pulmonary CT (computed tomography) scan, etc. The terms "lung scan" and "pulmonary scan" are synonyms and are used interchangeably herein. Similarly, the terms "lung nodule" and "pulmonary nodule" are used interchangeably.

As explained above, a lung nodule may indicate cancer but may equally well be related to a non-cancer disorder, i.e. lung nodules are often indeterminate. The inventive method combines imaging information and laboratory diagnostic information to manage indeterminate pulmonary nodules, i.e. to arrive at a reliable assessment (e.g. a risk score) for a pulmonary nodule. An advantage of the inventive method is that it reliably distinguishes between benign and malignant lung nodules. By applying machine learning, an AI classifier is able to identify information in the lung scan and blood panel and to combine this information to arrive at an accurate assessment of the lung nodule.

According to the invention, the method of training a classifier for use in the inventive lung nodule assessment method comprises the steps of
A) annotating a lung scan to identify a lung nodule and a number of image markers associated with the lung nodule;
B) annotating a blood panel to identify a number of blood markers associated with lung cancer;
C) determining an assessment for the lung nodule for use as a ground truth by the classifier;
D) applying the classifier to the lung scan, the blood panel and the associated ground truth; and
repeating steps A - D until a desired level of accuracy has been achieved.

According to the invention, the lung nodule assessment arrangement comprises an imaging module configured to provide a lung scan of a patient; a blood analyser configured to provide a blood panel for that patient; a processing unit configured to perform steps of the lung nodule assessment method when the classifier trained according to the inventive training method loaded into a memory of the processing unit; and a user interface configured at least to display the lung nodule assessment result. The "lung nodule assessment method" may simply be referred to as the "assessment method" in the following.

The object of the invention is also achieved by a computer program product comprising a computer program that is directly loadable into a memory of a control unit of a lung nodule assessment arrangement and which comprises program elements for performing steps of the inventive lung nodule assessment method when the computer program is executed by the control unit.

The object of the invention is also achieved by a computer-readable medium on which is stored program elements that can be read and executed by a computer unit in order to perform steps of the inventive lung nodule assessment method when the program elements are executed by the computer unit.

Particularly advantageous embodiments and features of the invention are given by the dependent claims, as revealed in the following description. Features of different claim categories may be combined as appropriate to give further embodiments not described herein.

In the following, it may be assumed that the lung scan is obtained from a high-resolution imaging apparatus such as a CT scanner. A blood panel can be provided in the form of a machine-readable file, for example by a laboratory equipped to analyse blood samples. The lung scan and blood panel are preferably obtained at about the same time, for example within a few weeks.

The classifier is trained with a training cohort preferably comprising at least 100 datasets, more preferably comprising at least 1,000 datasets, most preferably comprising at least 10,000 datasets. A dataset shall be understood to comprise at least one lung scan of a patient and at least one blood panel for that patient. A training dataset can also comprise one or more older lung scans and one or more older blood scans for the same patient.

The methodology for processing an input dataset is explained in the following: trained personnel such as radiologists or other clinicians examine images obtained from lung scans (e.g. CT, MRI, ultrasound, photoacoustic tomography, diffusion-weighted tomography, positron emission tomography scans) to identify lung nodules. Any lung nodule visible in an image is annotated in a suitable manner, e.g. a contour can be drawn about the nodule in each image of a sequence (in the case of a 3D image such as a CT scan). The clinician preferably also provides additional annotations regarding the aetiology of the nodule, for example "nodule is a carcinoma", "lung nodule is a hydatid cyst", etc. A global annotation such as "nodule is malignant" or "nodule is benign" is also preferably added. In a similar fashion, annotations are added to the blood panel accompanying each lung scan. Relevant blood markers may be protein markers (hormones, enzymes, antibodies), genomic markers, epigenomic markers, transcript RNA markers, microbiomic markers, etc. An annotation is preferably made for each blood marker that is above or below a critical threshold, i.e. a threshold that is critical in the context of lung cancer.

This step of annotating the image data and blood panel converts the datasets into training data that is relevant to the objective of assessing the nature of a lung nodule in terms of its aetiology and severity. For each dataset, the clinician can formulate an assessment for the lung nodule visible in the image data, for example a risk score, a malignancy probability, etc. This assessment is a ground truth for the classifier.

A lung nodule can appear as a small dark region within the lung. Various characteristics can be relevant in the assessment of its nature. Therefore, in a preferred embodiment of the invention, the classifier is configured to extract or retrieve a number of image markers from the lung scan. An image marker can comprise any of: vascular convergence; pleural indentation; air bronchogram; calcification type; cavitation; endobronchial origin; perifissural location; subpleural location; morphology; spiculation, lobulation, associated cystic airspace, bubble-like lucencies in a nodule. Preferably, the classifier is configured to perform image segmentation, for example semantic or context-driven image segmentation, to identify any such image markers in the lung scan.

It is known that certain pulmonary comorbidities such as COPD (chronic obstructive pulmonary disease) increase the risk of developing lung cancer. Therefore, in a particularly preferred embodiment of the invention, the classifier automatically identifies image markers associated with a respiratory disease such as emphysema, airway inflammation, pleural effusion, interstitial lung disease, infections, vascular disease, etc. The number of identified image markers and the relevance of each image marker can be used by the classifier to arrive at a more precise risk score for a lung nodule.

In a patient with lung cancer, physical changes may be observed to the lung over time. An early indicator of pulmonary disease is a reduction in size of a pulmonary volume such as lobar volume or a segmental volumes. Therefore, in a further preferred embodiment of the invention, the classifier is configured to identify a discrepancy between the current lung scan and an older lung scan of that patient. Particularly in the case of a patient participating in a lung cancer screening program, multiple older lung scans may be available for that patient. For example, the classifier may be configured to determine the volume of a specific pulmonary region in the most recent lung scan, to determine the volume of the same pulmonary region in one or more older lung scans for the same patient, and to compare the results. Any reduction in lung volume over time can be included by the classifier in its assessment of a lung nodule.

A lung nodule can be detected incidentally, for example, a lung nodule may be noticed in a CT image generated for a different reason, for example to assess injury following an accident. Alternatively, a lung nodule made be identified in the course of a lung cancer screening program. The probability for cancer is somewhat different in both scenarios, since a patient that participates in a cancer screening program generally already carries a higher risk of developing cancer. Screening-compliant patients can have annual scans, thereby reducing the risk of missing cancer as it can still be detected on the next scan. In contrast, incidentally found lung nodules may be followed by a more aggressive patient management approach in some countries, since the patient might not undergo further scanning. In such cases, the correct assessment of an incidentally-discovered lung nodule is very important because any decision to rule out cancer has to be made with high degree of certainty. Therefore, in a particularly preferred embodiment of the invention, the classifier is trained with a first training cohort in which the datasets are obtained from participants in lung cancer screening procedures; and with a second training cohort in which the datasets are obtained from patients undergoing incidental lung scans. This "two-pronged" training allows the classifier to be configured in a context-specific manner in order to deal with these different scenarios. For example, the classifier can have slightly different operating points or decision thresholds for "screening nodules" and "incidental nodules", i.e. any decision thresholds for a lung nodule identified in the course of a screening program may differ slightly from decision thresholds for a lung nodule identified incidentally.

It is known that certain biomarkers, for example certain blood markers, are elevated in only some types of lung cancer, possibly only in the case of one specific type of lung cancer. Furthermore, it is known that nodules resulting from different types of lung cancer develop in specific locations within the lung. For example, lung nodules associated with some types of lung cancer develop in central locations, while nodules associated with other types of lung cancer develop in peripheral locations. Therefore, in a further preferred embodiment of the invention, classifier is configured to identify the type of cancer associated with a lung nodule detected in the lung scan. For example, the classifier can be configured to determine whether the lung nodule is indicative of small-cell carcinoma, adenocarcinoma, squamous cell carcinoma, large cell carcinoma, etc.

Some blood markers such as autoantibodies are not specific to lung cancer but may also be elevated in the case of other non-pulmonary cancers such as prostate cancer, gastric cancer, liver cancer, testicular cancer, breast cancer, colorectal cancer, etc. Furthermore, such blood markers may also be elevated in a patient with an autoimmune disease. Therefore, in a particularly preferred embodiment of the invention, the classifier is trained to perform lung nodule evaluation also on the basis of extrapulmonary image data. In addition to current lung scan(s) and blood panel(s) for each patient, a training dataset can also comprise one or more extrapulmonary scans. The inventive lung nodule assessment method preferably comprises a preparatory step of obtaining extrapulmonary image data. For example, image markers from a mammography may indicate an advanced stage of breast cancer, and it may be assumed that the lung nodule is not the cause of the elevated blood markers. Equally, the risk score of a lung nodule may be higher if the image markers of a mammography indicate only a very minor lesion. In this way, the classifier is trained and configured to assess a lung nodule not only in the context of imaging markers from the lung scan and blood markers from a blood panel, but also in the context of markers from extrapulmonary images. An advantage is that the specificity and assessment accuracy of the inventive method can be favourably high.

The lung nodule assessment method may conclude with a report stating the outcome. For example, if the classifier has detected a lung nodule in the lung scan, it may report a risk score for that lung nodule. The risk score can be a Solitary Pulmonary Nodule (SPN) malignancy risk score based on the Mayo Clinic Model, on the Bayesian Inference Malignancy Calculator, or any such well-established model. A suitable approach to patient management may depend on the risk score, on the probable nature of the carcinoma, on other illnesses of the patient, etc. Therefore, in a preferred embodiment of the invention, the classifier may also report the cancer type associated with the lung nodule, for example in terms of probability. In a preferred embodiment of the invention, the classifier proposes a suitable patient management procedure on the basis of the lung nodule assessment, for example to schedule a biopsy, a PET scan, CT surveillance, etc. If adjuvant radiation treatment or preventive radiation treatment is proposed, the classifier may also compute a suitable Gray dose for a specific lung nodule. These various types of information can form part of a report that is generated for the clinician or user.

A lung nodule seen in an image can be characterized by various features such as its location, degree of vascular convergence, degree of pleural indentation, etc. Manual determination of such features or image markers can be tedious and error-prone. Therefore, in a particularly preferred embodiment of the invention, the classifier automatically identifies or retrieves less dominant malignant nodule image markers or features such as vascular convergence, pleural indentation, air bronchograms, types of calcifications, cavitation, endobronchial origin, perifissural location, subpleural location, etc. The prior art approaches primarily strive to quantify the malignancy of a lung nodule and to express this as the risk score. However, it is known that a certain proportion of lung cancers is found post-mortem, i.e. such patients died not because of lung cancer, but with lung cancer. It is possible for such a patient to have had lung cancer with one or more nodules that grow only slowly, i.e. the nodules are indolent. Therefore, in a further preferred embodiment of the invention, the classifier quantifies the indolence of a lung nodule and includes the lung nodule indolence in computation of the risk score.

For example, the classifier can be trained with outcome data indicative of the level of aggressiveness. To this end, the rate of growth of a nodule can be determined by comparing annual lung scans of the same patient.

In a further preferred embodiment of the invention, outcome data is determined and provided to the classifier. For example, in the case of an aggressive nodule, the patient outcome and/or the degree of metastasis may be known. Such outcome data is preferably used in the training stage of the classifier. In this way, the inventive lung assessment arrangement can assist in deciding whether or not aggressive management is appropriate for a patient with an indolent (malignant but slow-growing) lung nodule. Under consideration of the relevant factors such as the measure of indolence, the patient's age, degree of metastasis etc., a more favourable approach to treatment may be established. By also including an assessment of the indolence of a lung nodule, the inventive assessment method has a significantly higher clinical utility than the prior art approaches.

Other objects and features of the present invention will become apparent from the following detailed descriptions considered in conjunction with the accompanying drawings. It is to be understood, however, that the drawings are designed solely for the purposes of illustration and not as a definition of the limits of the invention.
Figure 1 is a simplified block diagram to illustrate the invention;
Figure 2 is a simplified block diagram of an exemplary embodiment of the inventive lung nodule assessment arrangement;
Figure 3 is a simplified block diagram of a further embodiment of the inventive lung nodule assessment arrangement;
Figure 4 illustrates a training stage of the classifier of the inventive lung nodule assessment arrangement;
Figure 5 illustrates factors of relevance to lung cancer;
Figure 6 is an exemplary pulmonary scan showing a lung nodule.

In the diagrams, like numbers refer to like objects throughout. Objects in the diagrams are not necessarily drawn to scale.

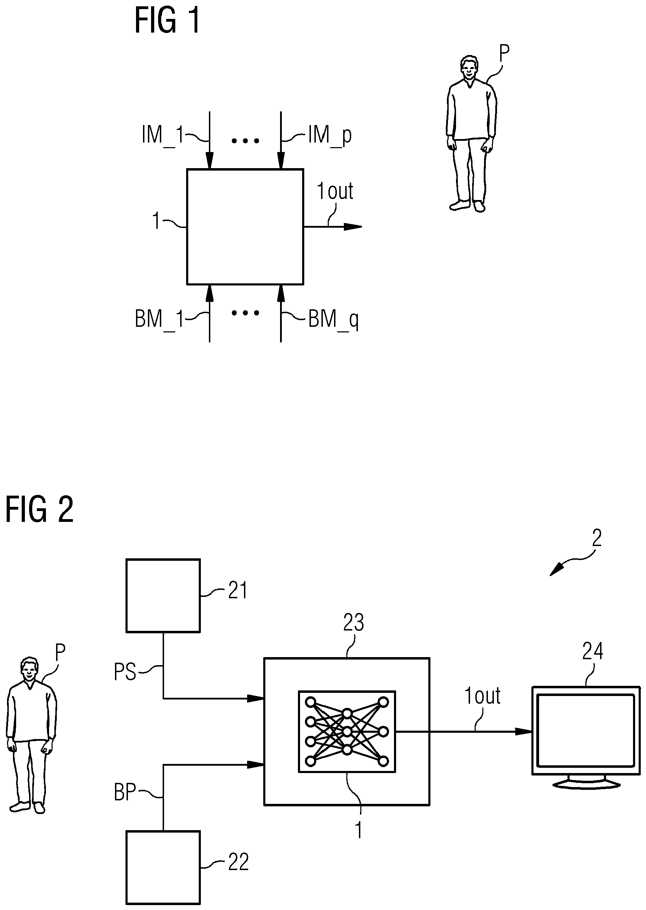

Figure 1 is a simplified block diagram to illustrate the concept underlying the invention. The diagram shows an AI classifier 1 such as a deep learning neural network, a Bayes classifier, a decision tree, etc. The classifier 1 receives a plurality of image markers IM_1, ..., IM_p and a plurality of blood markers BM_1, ..., BM_p. The image markers IM_1, ..., IM_p are obtained from a lung scan for a patient P. A clinician may already have identified a nodule in the lung scan. The blood markers BM 1, ..., BM p are obtained from analysis of a blood panel resulting from a blood sample of the same patient P. The classifier 1 has been trained to arrive at a diagnosis or assessment 1ₒᵤₜ. The assessment 1ₒᵤₜ can be in the form of a risk score, for example. In the case of a high risk score, i.e. the classifier 1 has concluded that a lung nodule is probably indicative of cancer, the assessment 1ₒᵤₜ may also include the likelihood of the lung nodule being related to a certain cancer type. The assessment 1ₒᵤₜ may be in the form of a more extensive report, and may also include a proposed cancer management plan for the patient if the risk score is above a certain threshold.

Figure 2 shows a simplified block diagram of an exemplary embodiment of the inventive lung nodule assessment arrangement 2, and shows an imaging modality 21 configured to provide a pulmonary scan PS of a patient P, and a blood analysis means 22 for providing a blood panel BP for that patient P. The lung nodule assessment arrangement 2 also comprises a processing unit 23 configured to perform steps of the method according to any of claims 1 to 9 when the trained classifier 1 is loaded into a memory of the processing unit.

A user interface 24 is configured to display the lung nodule assessment result 1ₒᵤₜ in the context of the pulmonary scan PS.

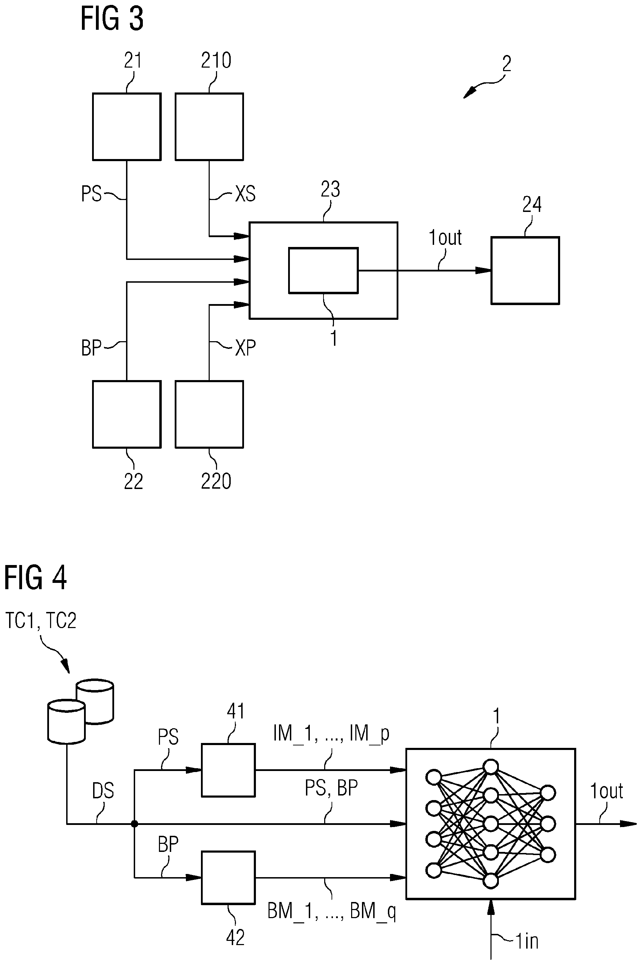

Figure 3 shows a simplified block diagram of another embodiment of the inventive lung nodule assessment arrangement 2. In addition to the pulmonary scan PS obtained from the imaging modality 21, an extrapulmonary scan XS may be obtained from a further imaging modality 210. In addition to the blood panel BP, a further set of biomarkers, e.g. salivary biomarkers, volatile organic compound (VOC) biomarkers from exhaled breath, urine biomarkers, stool biomarkers etc. may be obtained from a further biomarker modality 220. The trained classifier 1 processes the biomarkers from each source to arrive at an assessment for a lung nodule in the pulmonary scan PS. Here also, a user interface 24 displays the assessment result 1ₒᵤₜ in the context of the pulmonary scan PS.

The skilled person can be expected to choose a suitable classifier with the aim of processing image data and blood panel data, and can be assumed to be familiar with the procedure of training such a classifier. Figure 4 illustrates an exemplary training procedure for the classifier 1 of Figures 1 - 3. The classifier 1 in this exemplary embodiment is a neural network, illustrated in the conventional manner with an input layer, hidden layer and output layer each comprising a number of nodes. The nodes are connected according to coefficients or weights.

A dataset DS is provided, comprising a pulmonary scan PS and a blood panel BP of a specific patient. Both the pulmonary scan PS and the blood panel BP are annotated by experienced personnel to identify (in stage 41) image markers IM_1, ..., IM_p relevant to a lung nodule present in the pulmonary scan and to identify (in stage 42) blood markers BM_1, ..., BM_p relevant to lung cancer. For example, a clinician can determine image markers regarding the size, position and morphology of a lung nodule, and can also identify relevant elevations of proteins in the blood panel. The clinician computes an expected risk score for that lung nodule. Other relevant markers present in the dataset - for example patient's age, gender, smoking history, comorbidities etc. - are also included in the annotated dataset fed into the classifier 1. The clinician's assessment, e.g. an estimated risk score for the lung nodule, is a ground truth 1ᵢₙ for the classifier. A training cohort can comprise many hundreds or even thousands of datasets. A dataset for a patient may also include lung scans taken at intervals, for example annually. A dataset for a patient may also include an electronic health record (EHR) or electronic medical record (EMR), for example. In addition to any recent lung scan and blood panel, an electronic record can comprise older lung scans as well as older blood panels, and may of course include any other type of scan, laboratory results, etc.

The annotated datasets are used to train a classifier (regression model, deep learning model, etc.) to distinguish between benign and malignant nodules, indolent and aggressive nodules, etc. The pulmonary scan PS, the blood panel BP, the markers IM_1, ..., IM_p, BM_1, ..., BM_p and the ground truth of each dataset are fed to the classifier 1, which learns to analyse image data and blood panel data with a view to assessing a lung nodule visible in the image data.

The classifier 1 can be trained using a single training cohort. However, more accurate and useful lung nodule assessment is obtained by training the classifier using a first training cohort TC1 comprising datasets (DS) obtained from lung cancer screening procedures, and also with a second training cohort TC2 in which the datasets DS comprise lung scans PS obtained incidentally.

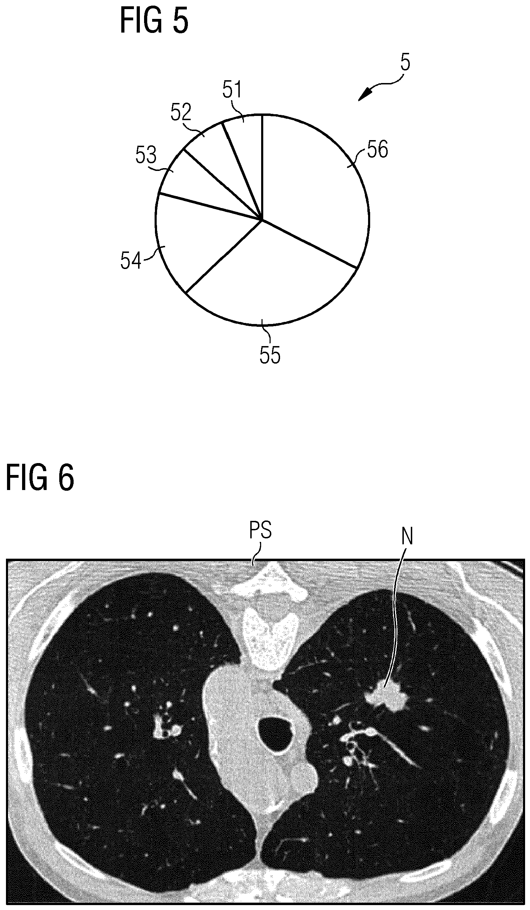

Figure 5 shows a pie chart that illustrates the relevance of different types of risk factor when assessing a pulmonary nodule. The location of the lung nodule, the presence of spiculations, and the patient's smoking history are risk factors 51, 52, 53 respectively, each contributing 7.3%. The patient's age is a risk factor 54 that contributes 18%. The nodule size is a more relevant risk factor 55 and contributes 30%. After determining the presence of a pulmonary nodule in a lung scan, a blood test is carried out for that patient. The remaining risk factor 56 is derived from relevant blood markers, for example the levels of relevant plasma proteins such as LG3BP and C163A, and contributes the final 30%. Conventional approaches that are based on image analysis do not include data from blood tests and therefore fail to consider valuable information relating to risk factor 56 (elevation levels of relevant blood biomarkers). Similarly, an assessment approach that is based on blood panel analysis does not consider valuable information relating to risk factor 55 (nodule size).

Figure 6 is an exemplary pulmonary scan showing a lung nodule N, which appears as a small mass. Many disorders lead to the development of benign or harmless lung nodules. However, lung cancer is always associated with the development of tumours which can start as small nodules as shown here. It is therefore very important to be able to correctly identify a lung nodule, i.e. to avoid a false negative diagnosis and also to avoid a false positive diagnosis.

Although the present invention has been disclosed in the form of preferred embodiments and variations thereon, it will be understood that numerous additional modifications and variations could be made thereto without departing from the scope of the invention.

For the sake of clarity, it is to be understood that the use of "a" or "an" throughout this application does not exclude a plurality, and "comprising" does not exclude other steps or elements. The mention of a "unit" or a "module" does not preclude the use of more than one unit or module.

## Claims

1. A method of performing lung nodule assessment, which method comprises the steps of
- obtaining a lung scan (PS) for a patient (P) from an imaging modality (21);
- obtaining a blood panel (BP) for that patient (P) from a blood analysis modality (22);
- processing the lung scan (PS) and the blood panel (BP) in a classifier (1), which classifier (1) is trained to assess a lung nodule (N) on the basis of the lung scan (PS) and the blood panel (BP).

2. A method according to the preceding claim, wherein the classifier (1) is configured to identify a number of image markers (IM_1, ..., IM_p) from the lung scan (PS), an image marker (IM_1, ..., IM_p) comprising any of: vascular convergence; pleural indentation; air bronchogram; calcification type; cavitation; endobronchial origin; perifissural location; subpleural location; morphology; spiculation, lobulation, associated cystic airspace, bubble-like lucencies in a nodule.

3. A method according to any of the preceding claims, wherein the classifier (1) is configured to identify a number of blood markers (BM_1, ..., BM_p) from the blood panel (BP), a blood marker (BM_1, ..., BM_p) comprising any of: a genomic marker, an epigenomic marker, a transcriptomic marker, a metabolomics marker, a proteomic marker.

4. A method according to any of the preceding claims, wherein the classifier (1) is configured to identify a relationship between an image marker (IM_1, ..., IM_p) and a respiratory disease.

5. A method according to any of the preceding claims, wherein the classifier (1) is configured to identify a discrepancy between the lung scan (PS) and an older lung scan of that patient (P).

6. A method according to the preceding claim, wherein the classifier (1) is configured to compare the volume of a specific region in the lung scan (PS) to the volume of the same region in the older lung scan (PS) and/or to quantify the indolence of a lung nodule (N) identified in the lung scan (PS).

7. A method according to the preceding claim, wherein coefficients of the classifier (1) are chosen on the basis of the context in which the lung scan (PS) was obtained.

8. A method according to any of the preceding claims, wherein the classifier (1) is configured to propose a patient management procedure on the basis of the lung nodule assessment.

9. A method according to any of the preceding claims, wherein the classifier (1) is trained to perform lung nodule assessment also on the basis of extrapulmonary image data (XS), and wherein the method comprises a preparatory step of obtaining extrapulmonary image data (XS).

10. A method of training a classifier (1) for use in the method according to any of claims 1 to 9, which method comprises the steps of
A) annotating a lung scan (PS) to identify a lung nodule (N) and a number of image markers (IM_1, ..., IM_p) associated with the lung nodule (N);
B) annotating a blood panel (BP) to identify a number of blood markers (BM_1, ..., BM_p) associated with lung cancer;
C) determining a assessment (1ᵢₙ) for the lung nodule (N) for use as a ground truth by the classifier (1);
D) applying the classifier (1) to the lung scan (PS), the blood panel (BP) and the associated ground truth (1ᵢₙ); and
repeating steps A - D until a desired level of accuracy has been achieved.

11. A method according to the preceding claim, wherein the classifier (1) is trained with a training cohort comprising at least 100 datasets (DS), more preferably at least 1,000 datasets (DS), most preferably at least 10,000 datasets (DS), wherein a dataset (DS) comprises at least one lung scan (PS) of a patient (P) and at least one blood panel (BP) for that patient (P).

12. A method according to claim 10 or claim 11, wherein the classifier (1) is trained with a first training cohort (TC1) in which the datasets (DS) comprise lung scans (PS) obtained from lung cancer screening procedures; and with a second training cohort (TC2) in which the datasets (DS) comprise lung scans (PS) obtained incidentally.

13. A lung nodule assessment arrangement (2) comprising
- an imaging modality (21) configured to provide a lung scan (PS) of a patient (P);
- a blood analysis modality (22) configured to provide a blood panel (BP) for that patient (P);
- a processing unit (23) configured to perform steps of the method according to any of claims 1 to 9 when the classifier (1) trained according to any of claims 10 to 12 is loaded into a memory of the processing unit (23); and
- a user interface (24) configured at least to display the lung nodule assessment result (1ₒᵤₜ) in the context of the lung scan (PS).

14. A computer program product comprising a computer program that is directly loadable into a memory of a control unit of a lung nodule assessment arrangement (2) according to claim 13 and which comprises program elements for performing steps of the method according to any of claims 1 to 9 when the computer program is executed by the control unit of the lung nodule assessment arrangement (2).

15. A computer-readable medium on which is stored program elements that can be read and executed by a computer unit in order to perform steps of the method according to any of claims 1 to 9 when the program elements are executed by the computer unit.

## Amended claims

### Amended claims in accordance with Rule 137(2) EPC.

1. A method of performing lung nodule assessment, which method comprises the steps of
- obtaining a lung scan (PS) for a patient (P) from an imaging modality (21);
- obtaining a blood panel (BP) for that patient (P) from a blood analysis modality (22);
- processing the lung scan (PS) and the blood panel (BP) in a classifier (1), which classifier (1) is trained to assess a lung nodule (N) on the basis of the lung scan (PS) and the blood panel (BP),
wherein the classifier (1) is configured to compare the volume of a specific region in the lung scan (PS) to the volume of the same region in an older lung scan (PS) and/or to quantify the indolence of a lung nodule (N) identified in the lung scan (PS).

2. A method according to the preceding claim, wherein the classifier (1) is configured to identify a number of image markers (IM_1, ..., IM_p) from the lung scan (PS), an image marker (IM_1, ..., IM_p) comprising any of: vascular convergence; pleural indentation; air bronchogram; calcification type; cavitation; endobronchial origin; perifissural location; subpleural location; morphology; spiculation, lobulation, associated cystic airspace, bubble-like lucencies in a nodule.

3. A method according to any of the preceding claims, wherein the classifier (1) is configured to identify a number of blood markers (BM_1, ..., BM_p) from the blood panel (BP), a blood marker (BM_1, ..., BM_p) comprising any of: a genomic marker, an epigenomic marker, a transcriptomic marker, a metabolomics marker, a proteomic marker.

4. A method according to any of the preceding claims, wherein the classifier (1) is configured to identify a relationship between an image marker (IM_1, ..., IM_p) and a respiratory disease.

5. A method according to any of the preceding claims, wherein the classifier (1) is configured to identify a discrepancy between the lung scan (PS) and an older lung scan of that patient (P).

6. A method according to the preceding claim, wherein coefficients of the classifier (1) are chosen on the basis of the context in which the lung scan (PS) was obtained.

7. A method according to claim 6, wherein the classifier (1) has different operating points or decision thresholds for lung cancer screening procedures and incidental lung scans.

8. A method according to any of the preceding claims, wherein the classifier (1) is configured to propose a patient management procedure on the basis of the lung nodule assessment.

9. A method according to any of the preceding claims, wherein the classifier (1) is trained to perform lung nodule assessment also on the basis of extrapulmonary image data (XS), and wherein the method comprises a preparatory step of obtaining extrapulmonary image data (XS).

10. A method of training a classifier (1) for use in the method according to any of claims 1 to 9, which method comprises the steps of
A) annotating a lung scan (PS) to identify a lung nodule (N) and a number of image markers (IM_1, ..., IM_p) associated with the lung nodule (N);
B) annotating a blood panel (BP) to identify a number of blood markers (BM_1, ..., BM_p) associated with lung cancer;
C) determining a assessment (1ᵢₙ) for the lung nodule (N) for use as a ground truth by the classifier (1);
D) applying the classifier (1) to the lung scan (PS), the blood panel (BP) and the associated ground truth (1ᵢₙ); and
repeating steps A - D until a desired level of accuracy has been achieved, wherein the classifier (1) is configured to compare the volume of a specific region in the lung scan (PS) to the volume of the same region in the older lung scan (PS) and/or to quantify the indolence of a lung nodule (N) identified in the lung scan (PS).

11. A method according to the preceding claim, wherein the classifier (1) is trained with a training cohort comprising at least 100 datasets (DS), more preferably at least 1,000 datasets (DS), most preferably at least 10,000 datasets (DS), wherein a dataset (DS) comprises at least one lung scan (PS) of a patient (P) and at least one blood panel (BP) for that patient (P).

12. A method according to claim 10 or claim 11, wherein the classifier (1) is trained with a first training cohort (TC1) in which the datasets (DS) comprise lung scans (PS) obtained from lung cancer screening procedures; and with a second training cohort (TC2) in which the datasets (DS) comprise lung scans (PS) obtained incidentally.

13. A lung nodule assessment arrangement (2) comprising
- an imaging modality (21) configured to provide a lung scan (PS) of a patient (P);
- a blood analysis modality (22) configured to provide a blood panel (BP) for that patient (P);
- a processing unit (23) configured to perform steps of the method according to any of claims 1 to 9 when the classifier (1) trained according to any of claims 10 to 12 is loaded into a memory of the processing unit (23); and
- a user interface (24) configured at least to display the lung nodule assessment result (1ₒᵤₜ) in the context of the lung scan (PS).

14. A computer program product comprising a computer program that is directly loadable into a memory of a control unit of a lung nodule assessment arrangement (2) according to claim 13 and which comprises program elements for performing steps of the method according to any of claims 1 to 9 when the computer program is executed by the control unit of the lung nodule assessment arrangement (2).

15. A computer-readable medium on which is stored program elements that can be read and executed by a computer unit in order to perform steps of the method according to any of claims 1 to 9 when the program elements are executed by the computer unit.
